Europäisches Patentamt

European Patent Office

Office européen des brevets

(19)

(11) **EP 0 927 886 B1**

(12) **FASCICULE DE BREVET EUROPEEN**

(45) Date de publication et mention
de la délivrance du brevet:
**17.09.2003 Bulletin 2003/38**

(51) Int Cl.[7]: **G01N 33/28**, G01N 21/64

(21) Numéro de dépôt: **98403301.9**

(22) Date de dépôt: **24.12.1998**

(54) **Procédé de détection et de caractérisation d'hydrocarbures de formation**

Verfahren zur Detektion und Charakterisierung von Kohlenwasserstoffen in unterirdischen Lagerstätten

Method of detecting and characterising hydrocarbons in underground formations

(84) Etats contractants désignés:
**DE ES GB IT NL**

(30) Priorité: **30.12.1997 FR 9716711**

(43) Date de publication de la demande:
**07.07.1999 Bulletin 1999/27**

(73) Titulaire: **Elf Exploration Production**
**92400 Courbevoie (FR)**

(72) Inventeurs:
• **Boehm, Claudine**
**64800 Beuste (FR)**

• **Berrut, Jean Bernard**
**64000 Pau (FR)**

(74) Mandataire: **Clisci, Serge et al**
**S.A. FEDIT-LORIOT**
**CONSEILS EN PROPRIETE INDUSTRIELLE**
**38, avenue Hoche**
**75008 Paris (FR)**

(56) Documents cités:
**EP-A- 0 342 869      GB-A- 2 142 955**
**US-A- 4 783 416      US-A- 4 814 614**
**US-A- 4 959 549**

**Description**

## DOMAINE TECHNIQUE

La présente invention concerne la recherche et l'exploitation d'hydrocarbures de formation.

Elle trouve son application dans les laboratoires d'analyses et sur les chantiers de forage de champs pétrolifères.

## ETAT DE LA TECHNIQUE ANTERIEURE

Les opérations de forage pour la recherche d'hydrocarbures dans les formations souterraines sont longues et coûteuses.

Un moyen pour réduire la durée et le coût de ces opérations est l'optimisation de la détection des hydrocarbures de formation dans toutes conditions de forage.

Cette optimisation consiste à s'assurer de la reconnaissance de toutes les roches imprégnées et à orienter le programme de test de manière à définir avec précision les zones à tester.

Le document EP 0 344 950 décrit une méthode pour déterminer la quantité d'hydrocarbures présents dans une formation souterraine, qui consiste à exécuter les étapes suivantes :

- prélever un échantillon de roche,
- extraire les hydrocarbures contenus dans une quantité connue de l'échantillon de roche, au moyen d'une quantité connue d'un solvant,
- exciter l'extrait obtenu par un rayonnement ultraviolet d'une longueur d'onde à laquelle la plupart des mélanges d'hydrocarbures sont fluorescents, comprise entre 250 et 310 nanomètres,
- enregistrer au moyen d'un fluorimètre, le rayonnement émis par l'extrait à une longueur d'onde donnée,
- déterminer la quantité d'éléments fluorescents présents dans l'échantillon de la roche en comparant l'intensité du rayonnement enregistré avec les rayonnements émis à la même longueur d'onde par des échantillons de matières contenant des quantités connues d'hydrocarbures.

L'échantillon de roche étant imprégné à la fois par les hydrocarbures de formation et les éléments fluorescents entrant dans la composition de la boue de forage, les résultats obtenus par cette méthode sont imprécis. De plus ils ne donnent aucune information sur la nature des éléments fluorescents détectés, tout particulièrement dans le cas des boues à l'huile.

## EXPOSE DE L'INVENTION

La présente invention a justement pour objet de remédier à ces inconvénients et de fournir un procédé de détection et de caractérisation des hydrocarbures de formation.

Ce procédé est utilisable en laboratoire et sur les chantiers de forages de champs pétrolifères

A cette fin la présente invention propose un procédé de détection et de caractérisation d'hydrocarbures de formation consistant à exécuter les étapes suivantes :

- prélever au cours d'un forage un échantillon de matière représentatif des roches traversées,
- prélever sur ledit échantillon de matière une quantité déterminée de matière à analyser,
- extraire les hydrocarbures contenus dans ladite quantité de matière à analyser, au moyen d'une quantité connue d'un solvant d'hydrocarbures pour constituer un extrait initial,

  caractérisé en ce qu'il consiste en plus à exécuter les opérations suivantes :

- diluer l'extrait initial d'un facteur d pour obtenir un extrait final,
- exciter l'extrait final au moyen d'un rayonnement ultraviolet d'une longueur d'onde choisie dans la bande comprise sensiblement entre 250 et 400 nanomètres,
- enregistrer dans une bande L de longueurs d'ondes, un spectre d'émission résultant de l'excitation de l'extrait final,
- calculer le flux d'émission de l'échantillon de matière par application de la formule suivante :

$$\text{FLUX} = d * \sum_{i=1}^{n} I(\lambda i)$$

dans laquelle

- . FLUX représente le flux d'émission de l'échantillon de matière,
- . I $(\lambda i)$ représente l'intensité du rayonnement émis à une longueur d'onde $\lambda i$ de la bande L par l'extrait final,
- . n représente le nombre de longueurs d'ondes du spectre d'émission échantillonné à un pas compris entre 1 et 20 nanomètres, il est égal à L divisée par le pas,
- . i varie de 1 à n.

la valeur du flux d'émission de l'échantillon de matière est proportionnelle à la concentration en éléments fluorescents de l'échantillon de matière dont notamment les hydrocarbures de formation,

- calculer le quotient de fluorescence de l'échantillon de matière au moyen de la formule suivante :

QF = Aire A / Aire B

dans laquelle :

QF représente le quotient de fluorescence de l'échantillon de matière,
Aire A et Aire B sont calculées respectivement par les formules suivantes :

$$\text{Aire A} = \sum_{i=x+1}^{n} I(\lambda i)$$

$$\text{Aire B} = \sum_{i=1}^{x} I(\lambda i)$$

dans lesquelles :

- . I $(\lambda i)$ et n sont définis comme précédemment,
- . x est un nombre entier compris entre 1 et n,

la valeur du quotient de fluorescence de l'échantillon de matière est représentative de la nature des hydrocarbures contenus dans l'échantillon de matière.

Selon une autre caractéristique du procédé de l'invention le solvant d'hydrocarbures est préférentiellement du cyclohexane.
Selon une autre caractéristique du procédé de l'invention la bande L de longueurs d'ondes est comprise de préférence sensiblement entre 280 et 500 nanomètres,
**[0013]** Selon une autre caractéristique du procédé de l'invention la valeur x correspond au repère d'une longueur d'onde $\lambda x$ sensiblement égale à 367 nanomètres, représentative de la coupure entre les spectres émis d'une part, par les hydrocarbures monoaromatiques, diaromatiques et une partie des triaromatiques et d'autre part, par les hydrocarbures polyaromatiques.
Selon une autre caractéristique du procédé de l'invention une valeur faible du quotient de fluorescence de l'échantillon de matière est significative de la présence d'hydrocarbures légers dans ledit échantillon et une valeur élevée est significative de la présence d'hydrocarbures lourds.
Selon une autre caractéristique le procédé de l'invention consiste à prélever une série d'échantillons de matière à des profondeurs différentes au cours du forage, à déterminer le quotient de fluorescence et le flux d'émission de chaque échantillon, puis à tracer les courbes représentatives dudit quotient de fluorescence et dudit flux d'émission en fonction des profondeurs, des variations du quotient de fluorescence et du flux d'émission, permettant respectivement de caractériser la nature des éléments fluorescents et d'apprécier l'importance de la concentration desdits éléments dans la série d'échantillons de matière.

Selon une autre caractéristique le procédé de l'invention consiste à comparer le quotient de fluorescence d'au moins un échantillon de matière, au quotient de fluorescence d'au moins un échantillon de produit de référence déterminé de la même manière que le quotient de fluorescence de l'échantillon de matière.

Selon une autre caractéristique du procédé de l'invention le forage étant effectué avec une injection de boue, l'échantillon de produit de référence est constitué par un échantillon de boue prélevé en cours de forage.

Selon une autre caractéristique du procédé de l'invention une valeur de quotient de fluorescence d'un échantillon de matière, proche de la valeur du quotient de fluorescence de l'échantillon de boue prélevé en cours de forage, est significative de l'absence d'hydrocarbures de formation dans l'échantillon de matière et une valeur différente, est significative d'une présence si de plus la valeur du flux d'émission de l'échantillon de matière est élevée.

## BREVE DESCRIPTION DES DESSINS

L'invention sera mieux comprise à l'aide de la description qui suit, d'un mode de réalisation donné à titre d'exemple et en référence aux dessins annexés dans lesquels :

- la figure 1 représente un spectre d'émission par fluorescence d'un extrait final obtenu à partir d'un échantillon de matière,
- la figure 2 représente un spectre d'émission par fluorescence d'un extrait final obtenu à partir d'un échantillon de matière, avec indication de la longueur d'onde $\lambda x$,
- la figure 3 est un diagramme qui représente les valeurs des flux d'émission d'une suite d'échantillons de matière en fonction de la profondeur de prélèvement desdits échantillons,
- la figure 4 est un diagramme qui représente les valeurs des quotients de fluorescence d'une suite d'échantillons de matière en fonction de la profondeur de prélèvement desdits échantillons,

## EXPOSE DETAILLE DE L'INVENTION

D'une manière générale le procédé de l'invention est utilisé pour détecter et caractériser des hydrocarbures de formation.

Ce procédé consiste à prélever à différentes profondeurs au cours d'un forage, une partie des déblais pour constituer une suite d'échantillons de matière, représentative des roches traversées.

Puis après séchage et broyage de chaque échantillon prélever une quantité déterminée de matière à analyser, à extraire au moyen d'une quantité connue de cyclohexane, les hydrocarbures contenus dans la quantité de matière à analyser issue de chaque échantillon pour constituer une suite d'extraits initiaux. Ces extraits initiaux sont ensuite dilués d'un facteur de dilution d pour obtenir des extraits finaux. Le facteur d est déterminé expérimentalement pour chaque échantillon de manière à ce que les concentrations en hydrocarbures dans les extraits finaux se situent dans le domaine de mesure du spectrofluorimètre qui sera utilisé par la suite.

L'utilisation du cyclohexane comme solvant des hydrocarbures est particulièrement intéressante car elle privilégie l'extraction des composés polyaromatisés représentatifs des hydrocarbures de formation récupérables alors que les solvants aliphatiques sont plus restrictifs et que les solvants chlorés dissolvent également les hydrocarbures lourds et polaires qui sont moins mobiles et donc plus difficilement récupérables.

Chaque extrait final est introduit dans un spectrofluorimètre où il est excité par un rayonnement ultraviolet de longueur d'onde égal à 265 nanomètres. Cette longueur d'onde est choisie en raison de la propriété connue qu'ont les hydrocarbures ainsi irradiés d'émettre par fluorescence. Cette longueur d'onde présente aussi l'avantage de maximiser l'émission par fluorescence des hydrocarbures de formation et de minimiser celle des hydrocarbures contenus dans les boues à l'huile.

L'intensité du rayonnement émis par fluorescence par chaque extrait final est enregistrée au moyen du spectrofluorimètre sur une bande L de longueurs d'ondes significatives comprises entre 288 et 498 nanomètres.

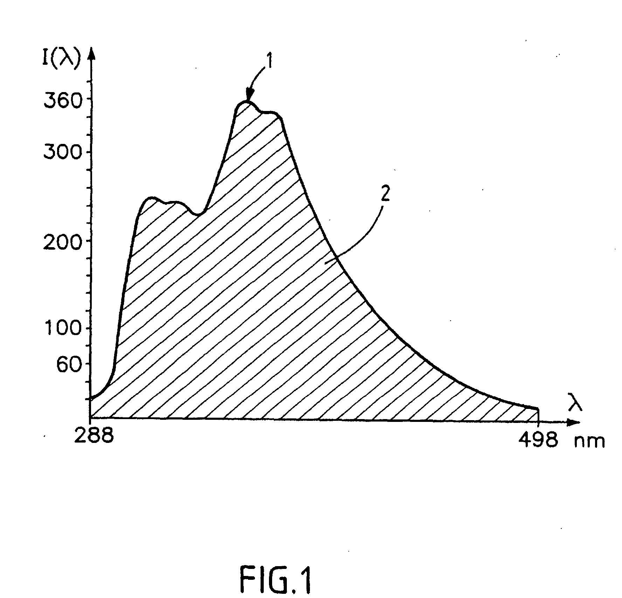

On obtient ainsi pour chaque extrait final un spectre $I(\lambda)$ tel que celui de la figure 1 qui représente les variations de l'intensité du rayonnement émis par fluorescence en fonction de la longueur d'onde $\lambda$ d'émission. La forme de ce spectre est la signature directe de la nature du fluide qui imprègne les roches de la zone dans laquelle l'échantillon de matière a été prélevé.

On calcule ensuite le flux d'émission de chaque échantillon de matière en appliquant la formule suivante :

$$FLUXp = d * \sum_{i=1}^{n} Ip(\lambda i)$$

dans laquelle :

- FLUXp représente le flux d'émission de l'échantillon prélevé à la profondeur p,
- Ip ($\lambda$i) représente l'intensité du rayonnement émis, par l'extrait final obtenu à partir de l'échantillon prélevé à la profondeur p, à une longueur d'onde $\lambda$i de la bande L comprise entre 288 et 498 nanomètres,
- n représente le nombre de longueurs d'ondes du spectre d'émission échantillonné à un pas de 1 nanomètre, il. est égal à la largeur de bande d'enregistrement du spectre soit 498 moins 288 divisée par le pas, soit 210,
- i varie de 1 à n.

La valeur FLUXp est proportionnelle à la concentration en éléments fluorescents dont notamment les hydrocarbures de formation, contenus dans l'échantillon de matière, prélevé à la profondeur p.

Sur le diagramme de la figure 1 la valeur du flux d'émission de l'échantillon de matière est représentée par l'aire 2 du spectre c'est à dire l'aire hachurée comprise entre la courbe I($\lambda$) et l'axe $\lambda$.

Il est important de noter que, si on ne considère que la seule valeur de FLUXp ainsi obtenue, on ne dispose d'aucune information sur la nature des éléments à l'origine du rayonnement émis par fluorescence et qu'il est donc impossible d'en déduire avec certitude qu'il s'agit d'hydrocarbures de formation.

On calcule ensuite le quotient de fluorescence de l'échantillon de matière en appliquant la formule suivante :

$$QFp = Aire\ Ap\ /\ Aire\ Bp$$

dans laquelle :

QFp représente le quotient de fluorescence de l'échantillon de matière prélevé à la profondeur p,
Aire A et Aire B sont calculées respectivement par les formules suivantes :

$$Aire\ Ap = \sum_{i=x+1}^{n} Ip(\lambda i)$$

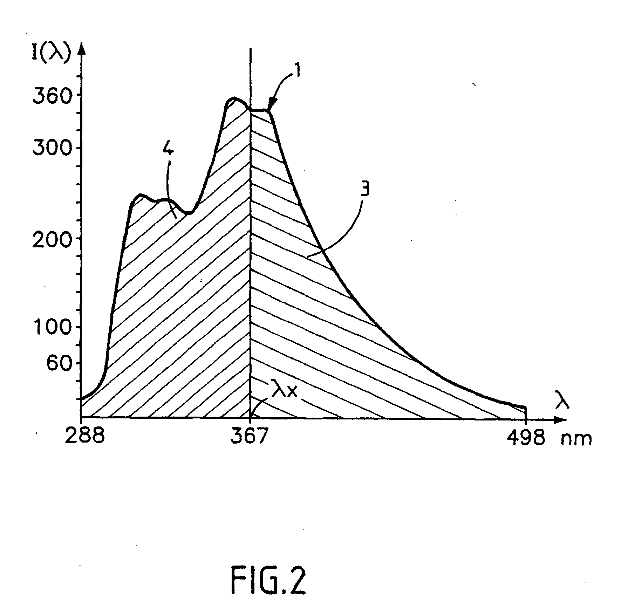

représentée par l'aire 3 sur la figure 2

$$Aire\ Bp = \sum_{i=1}^{x} Ip(\lambda i)$$

représentée par l'aire 4 sur la figure 2

dans lesquelles :

- Ip ($\lambda$i) et n sont définis comme précédemment,
- x est égal à 79; cette valeur correspond au repère de la longueur d'onde $\lambda$x = 367 nanomètres représentative de la coupure entre les spectres émis d'une part, par les hydrocarbures monoaromatiques, diaromatiques et une partie des hydrocarbures triaromatiques et d'autre part, par les hydrocarbures polyaromatiques dont une autre partie des hydrocarbures triaromatiques.

Le quotient de fluorescence de l'échantillon de matière est égal au rapport des aires 3 et 4 de la figure 2.

On détermine de la même manière le quotient QFb de fluorescence d'un échantillon de boue de forage prélevé au début du forage.

Si le quotient QFp de fluorescence d'un échantillon de matière est différent du quotient QFb de fluorescence de la boue et si la valeur FLUXp du flux d'émission du même échantillon est élevé, cela signifie que des hydrocarbures de formation sont présents à la profondeur p.

Si QFp est voisin de QFb et si la valeur FLUXp du flux d'émission du même échantillon est faible, cela signifie l'absence d'hydrocarbures de formation à la profondeur p.

Des valeurs voisines de quotients QFp de fluorescence obtenues à partir d'échantillons de matière prélevés à des profondeurs différentes signifient que les hydrocarbures détectés sont de natures voisines.

Des valeurs différentes de quotients QFp de fluorescence obtenues à partir d'échantillons de matières prélevés à des profondeurs différentes signifient que les hydrocarbures détectés à ces profondeurs sont de natures différentes.

Les valeurs des flux d'émission aux différentes profondeurs reflètent la concentration en hydrocarbures de formation présents dans les roches traversées.

Ainsi le procédé de l'invention permet de caractériser relativement la nature des éléments fluorescents extraits des échantillons de matières prélevés à différentes profondeurs et d'en apprécier la concentration dans toutes conditions de forage et particulièrement dans le cas des forages en boue à l'huile.

Grâce à l'invention on détecte toutes les zones traversées imprégnées par des hydrocarbures sans risque de confusion avec des zones non imprégnées.

Le mode de mise en oeuvre du procédé de l'invention qui est décrit ci-dessus est donné à titre d'exemple non limitatif, notamment en ce qui concerne le choix de la matière représentative des roches traversées au cours du forage, qui pourra avantageusement provenir de carottes ou de carottages latéraux.

EXEMPLE DE MISE EN OEUVRE DU PROCEDE DE L'INVENTION

On prélève au cours d'un forage en boue à l'eau, une suite de carottes représentatives des roches traversées.

Sur une carotte on prélève 1 gramme de matière qui constitue un échantillon de matière représentatif de la roche traversée à une profondeur donnée p. Cet échantillon est placé dans une coupelle en céramique et séché en étuve à 50°C pendant 30 minutes.

Il est ensuite broyé manuellement au moyen d'un mortier en agate.

On prélève ensuite 200 mg de l'échantillon de matière broyé qu'on place dans un tube à essai de 50 ml à bouchon de verre rodé.

Pour extraire les hydrocarbures contenus dans cette quantité ainsi déterminée de matière on verse 10 ml de cyclohexane dans le tube à essai qu'on agite manuellement pendant 20 secondes.

On laisse reposer l'ensemble matière broyée/cyclohexane pendant environ 10 minutes.

La partie liquide de cet ensemble constitue l'extrait initial d'hydrocarbures.

On dilue une partie de cet extrait initial d'un facteur d = 50 en ajoutant 10 ml de cyclohexane à 200 microlitres d'extrait filtré, obtenu au moyen d'une seringue de 1 ml munie à son extrémité d'un filtre jetable.

Cette solution constitue l'extrait final qu'on transfère en partie dans la cuve d'un spectrofluorimètre modèle LS50B fabriqué par la Société PERKIN ELMER.

L'extrait final est excité au moyen d'un rayonnement ultraviolet de longueur d'onde égale à 265 nanomètres de largeur de bande 15 nanomètres.

On enregistre le spectre brut du rayonnement émis par fluorescence par l'extrait final, c'est à dire qu'on enregistre les valeurs de l'intensité du rayonnement émis dans la bande L de longueur d'ondes 288 à 498 nm à la vitesse de 120 nm/minutes, au travers d'une fenêtre de largeur de bande 5 nm.

Le spectre brut enregistré est ensuite transféré sur un microcalculateur DIGITAL 466 relié au spectrofluorimètre.

Un blanc analytique est effectué en appliquant la même procédure que ci-dessus sans échantillon de matière.

Le spectre blanc ainsi obtenu est soustrait du spectre brut pour donner le spectre d'émission $I(\lambda i)$ à partir duquel on calcule le flux d'émission en appliquant la formule suivante :

$$FLUXp = dp * \sum_{i=1}^{n} Ip(\lambda i)$$

dans laquelle :

- FLUXp représente le flux d'émission de l'échantillon prélevé à la profondeur p,
- dp représente le facteur de dilution de l'extrait initial de l'échantillon prélevé à la profondeur p,
- Ip $(\lambda i)$ représente l'intensité du rayonnement émis par l'extrait final, à une longueur d'onde $\lambda i$ de la bande L comprise entre 288 et 498 nanomètres,
- n représente le nombre de longueurs d'ondes du spectre d'émission échantillonné à un pas de 1 nanomètre, il est égal à la largeur de bande d'enregistrement du spectre soit 498 moins 288 divisée par le pas, soit 210,
- i varie de 1 à n.

On calcule ensuite le quotient de fluorescence de l'échantillon prélevé à la profondeur p en appliquant la formule suivante :

$$QFp = Aire \; Ap \; / \; Aire \; Bp$$

dans laquelle :

QFp représente le quotient de fluorescence de l'échantillon de matière prélevé à la profondeur p,
Aire Ap et Aire Bp sont calculées respectivement par les formules suivantes :

$$Aire \; Ap = \sum_{i=x+1}^{n} Ip(\lambda i)$$

$$Aire \; Bp = \sum_{i=1}^{x} Ip(\lambda i)$$

dans lesquelles

. Ip ($\lambda$i) et n sont définis comme précédemment,
. x est égal à 79.

On répète cette suite d'opérations à partir d'échantillons prélevés à différentes profondeurs sur les carottes réalisées.
Pour les échantillons dont l'intensité maximale du rayonnement émis dépasse la valeur 800, la solution doit être de nouveau diluée.
Ainsi pour les échantillons p1 et p7 les facteurs de dilution sont respectivement de 167 et 125.
On obtient les résultats qui sont consignés dans le tableau ci-après :

TABLEAU 1

| Repère P | P en m | dp | FLUXp | Aire Ap a | Aire Bp b | QFp = a/b |
|----------|--------|-----|-----------|-----------|-----------|-----------|
| p0 | 1 785,20 | 50 | 395 700 | 4 239 | 3 675 | 1,15 |
| p1 | 2 019,85 | 167 | 3 988 127 | 15 252 | 8 629 | 1,77 |
| p2 | 2 020,70 | 50 | 2 212 750 | 28 098 | 16 157 | 1,74 |
| p3 | 2 023,18 | 50 | 781 900 | 9 814 | 5 823 | 1,69 |
| p4 | 2 025,50 | 50 | 40 400 | 455 | 353 | 1,29 |
| p5 | 2 027,35 | 50 | 2 569 150 | 32 535 | 18 848 | 1,73 |
| p6 | 2 028,33 | 50 | 3 211 850 | 42 807 | 21 430 | 2 |
| p7 | 2 032,37 | 125 | 5 962 000 | 31 644 | 16 052 | 1,97 |
| p8 | 2 033,40 | 50 | 2 029 400 | 26 238 | 14 351 | 1,83 |
| p9 | 2 036,35 | 50 | 39 450 | 476 | 313 | 1,52 |
| p10 | 2 038,47 | 50 | 1 250 | NS | NS | NS |
| p11 | 2 052,50 | 50 | 5 450 | 38 | 71 | 0,54 |
| p12 | 2 128,60 | 50 | 26 100 | 203 | 319 | 0,64 |
| p13 | 2 382,80 | 50 | 50 250 | 422 | 583 | 0,72 |
| p14 | 2 647,60 | 50 | 1 371 850 | 15 069 | 12 367 | 1,22 |

TABLEAU 1   (suite)

| Repère P | P en m | dp | FLUXp | Aire Ap a | Aire Bp b | QFp = a/b |
|----------|--------|-----|----------|-----------|-----------|-----------|
| p15 | 2 649,10 | 50 | 2 143 250 | 24 018 | 18 847 | 1,27 |
| p16 | 2 650,42 | 50 | 1 669 800 | 18 026 | 15 370 | 1,17 |
| p17 | 2 651,45 | 50 | 531 000 | 5 443 | 5 177 | 1,05 |
| p18 | 2 652,70 | 50 | 1 160 200 | 11 758 | 11 446 | 1,03 |
| p19 | 2 653,50 | 50 | 1 611 900 | 17 411 | 14 827 | 1,17 |
| p20 | 2 654,60 | 50 | 824 150 | 8 667 | 7 816 | 1,11 |
| p21 | 2 655,53 | 50 | 21 700 | 196 | 237 | 0,83 |
| p22 | 2 820,75 | 50 | 9 000 | 92 | 88 | 1,05 |

Dans ce tableau, P signifie profondeur à laquelle correspond le prélèvement de l'échantillon au cours du forage et NS valeur non significative.

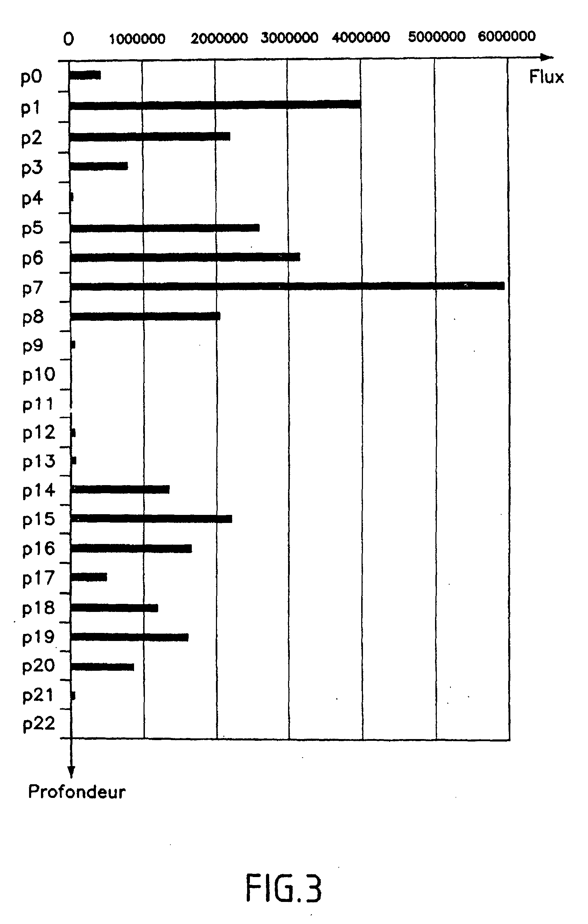

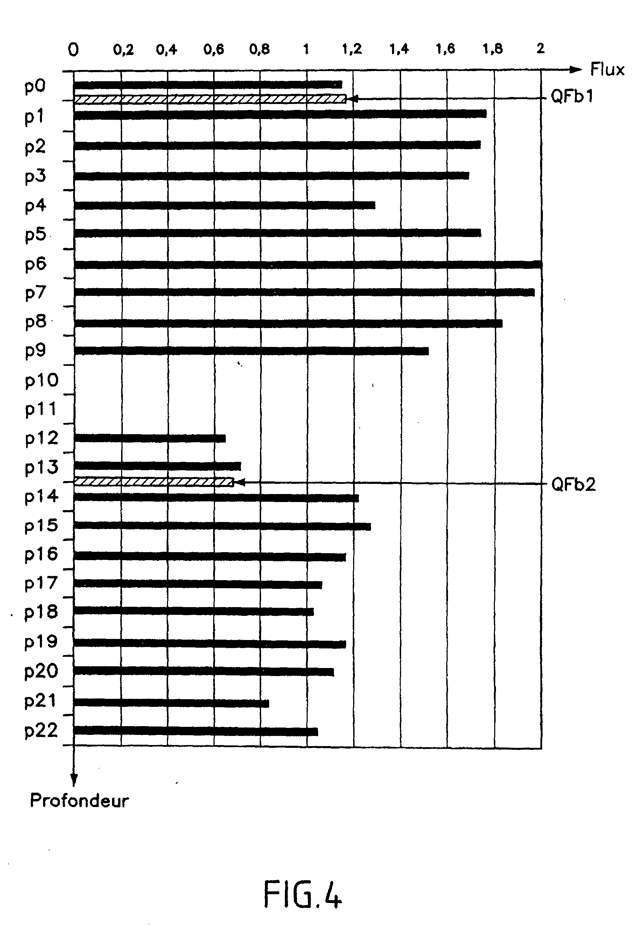

A partir de ces valeurs on trace les diagrammes des figures 3 et 4 qui représentent respectivement les valeurs des flux d'émission (FLUXp) et des quotients (QFp) de fluorescence des échantillons de matière en fonction des profondeurs auxquelles ont été prélevées les carottes.

Selon la même procédure on détermine le quotient de fluorescence de deux échantillons de produit de référence constitués par deux échantillons de boue prélevés au cours du forage lorsque ce dernier atteint les profondeurs de 1 790 mètres et 2 645 mètres.

On obtient ainsi :

Pour la profondeur de 1 790 m une valeur QFb1 = 1,18.
Pour la profondeur de 2 645 m une valeur QFb2 = 0,68.

Ces valeurs sont représentées sur la figure 3, respectivement entre les profondeurs repérées p0 et p1 d'une part et p13 et p14 d'autre part.

Le tableau ci-dessus et les diagrammes des figures 3 et 4 mettent en évidence deux zones A et B à intérêt pétrolier.

Les extraits finaux des échantillons prélevés dans la zone A délimitée par les profondeurs 2 019,85 m et 2 033,40 m sont caractérisés par :

- des valeurs de QFp comprises entre 1,69 et 2,00 qui sont élevées par rapport à la valeur 1,18 du quotient de fluorescence QFb1 de la boue de forage utilisée dans cette zone, sauf pour la profondeur de 2 025,50 m pour laquelle QFp vaut seulement 1,29.
- par des valeurs de flux (FLUXp) d'émission supérieures à 2 000 000 donc très élevées, sauf pour la profondeur de 2 023,18 m pour laquelle la valeur du flux est inférieure à 800 000 et pour la profondeur de 2 025,50 m pour laquelle la valeur du flux n'est que de 40 400 donc comparativement très faible.

Ces différences entre les valeurs des quotients de fluorescence des échantillons prélevés aux différentes profondeurs et la valeur du quotient de fluorescence de l'échantillon de boue sont significatives de la présence d'hydrocarbures de formation dans la zone A.

Les valeurs élevées des flux déterminées aux mêmes profondeurs indiquent que les hydrocarbures détectés sont présents en quantités importantes dans la zone A.

La très faible valeur du flux pour la profondeur de 2 025,50 m signifie que s'il y a des hydrocarbures à cette profondeur ils sont en très faible quantité.

Les échantillons prélevés dans la zone B délimitée par les profondeurs 2 647,60 m et 2 654,60 m sont caractérisés par :

- des valeurs de QFp comprises entre 1,03 et 1,27 qui sont élevées par rapport à la valeur 0,68 du quotient de fluorescence QFb2 de la boue de forage utilisée dans cette zone.
- par des valeurs de flux (FLUXp) d'émission supérieures à 1 000 000 donc élevées sauf pour les deux échantillons prélevés respectivement aux profondeurs p17 et p20 pour lesquels elles sont supérieures à 500 000.

Ces différences entre les valeurs des quotients de fluorescence des échantillons prélevés aux différentes profondeurs et la valeur du quotient de fluorescence de l'échantillon de boue sont significatives de la présence d'hydrocarbures de formation dans la zone B.

Les valeurs élevées des flux déterminées aux mêmes profondeurs indiquent que les hydrocarbures détectés sont présents en quantités importantes dans la zone B.

Les valeurs des flux de la zone A en moyenne plus élevées que celles des flux de la zone B reflètent une plus forte concentration en hydrocarbures dans la zone A que dans la zone B.

Les valeurs différentes des quotients QFp de fluorescence dans les zones A et B traduisent les natures différentes des hydrocarbures présents dans ces zones.

La supériorité des valeurs des quotients de fluorescence des échantillons de la zone A par rapport à celles des quotients de fluorescence de ceux de la zone B, indique que les hydrocarbures détectés dans la zone A sont plus lourds que ceux détectés dans la zone B.

## Revendications

**1.** Procédé de détection et de caractérisation d'hydrocarbures de formation consistant à exécuter les étapes suivantes :

- prélever au cours d'un forage un échantillon de matière représentatif des roches traversées,
- prélever sur ledit échantillon de matière une quantité déterminée de matière à analyser,
- extraire les hydrocarbures contenus dans ladite quantité de matière à analyser, au moyen d'une quantité connue d'un solvant d'hydrocarbures pour constituer un extrait initial,

**caractérisé en ce qu'**il consiste en plus à exécuter les opérations suivantes :

- diluer l'extrait initial d'un facteur d pour obtenir un extrait final,
- exciter l'extrait final au moyen d'un rayonnement ultraviolet d'une longueur d'onde choisie dans la bande comprise sensiblement entre 250 et 400 nanomètres,
- enregistrer dans une bande L de longueurs d'ondes, un spectre d'émission résultant de l'excitation de l'extrait final,
- calculer le flux d'émission de l'échantillon de matière par application de la formule suivante :

$$FLUX = d * \sum_{i=1}^{n} I(\lambda i)$$

dans laquelle

- . FLUX représente le flux d'émission de l'échantillon de matière,
- . $I(\lambda i)$ représente l'intensité du rayonnement émis à une longueur d'onde $\lambda i$ de la bande L par l'extrait final,
- . n représente le nombre de longueurs d'ondes du spectre d'émission échantillonné à un pas compris entre 1 et 20 nanomètres, il est égal à L divisée par le pas,
- . i varie de 1 à n.

la valeur du flux d'émission de l'échantillon de matière est proportionnelle à la concentration en éléments fluorescents de l'échantillon de matière dont notamment les hydrocarbures de formation,
- calculer le quotient de fluorescence de l'échantillon de matière au moyen de la formule suivante :

$$QF = Aire\ A\ /\ Aire\ B$$

dans laquelle :

QF représente le quotient de fluorescence de l'échantillon de matière,
Aire A et Aire B sont calculées respectivement par les formules suivantes :

$$\text{Aire } A = \sum_{i=x+1}^{n} I(\lambda i)$$

$$\text{Aire } B = \sum_{i=1}^{x} I(\lambda i)$$

dans lesquelles :

. $I(\lambda i)$ et n sont définis comme précédemment,
. x est un nombre entier compris entre 1 et n, la valeur du quotient de fluorescence de l'échantillon de matière est représentative de la nature des hydrocarbures contenus dans l'échantillon de matière.

**2.** Procédé selon la revendication 1 **caractérisé en ce que** le solvant d'hydrocarbures est préférentiellement du cyclohexane.

**3.** Procédé selon la revendication 1 ou 2 **caractérisé en ce que** la bande L de longueurs d'ondes est comprise de préférence sensiblement entre 280 et 500 nanomètres,

**4.** Procédé selon les revendications 1, 2 ou 3 **caractérisé en ce que** la valeur x correspond au repère d'une longueur d'onde $\lambda x$ sensiblement égale à 367 nanomètres, représentative de la coupure entre les spectres émis d'une part, par les hydrocarbures monoaromatiques, diaromatiques et une partie des triaromatiques et d'autre part, par les hydrocarbures polyaromatiques.

**5.** Procédé selon la revendication 4 **caractérisé en ce qu'** une valeur faible du quotient de fluorescence de l'échantillon de matière est significative de la présence d'hydrocarbures légers dans ledit échantillon et une valeur élevée est significative de la présence d'hydrocarbures lourds.

**6.** Procédé selon une des revendications 1 à 5 **caractérisé en ce qu'**il consiste à prélever une série d'échantillons de matière à des profondeurs différentes au cours du forage, à déterminer le quotient de fluorescence et le flux d'émission de chaque échantillon, puis à tracer les courbes représentatives dudit quotient de fluorescence et dudit flux d'émission en fonction des profondeurs, des variations du quotient de fluorescence et du flux d'émission, permettant respectivement de caractériser la nature des éléments fluorescents et d'apprécier l'importance de la concentration desdits éléments dans la série d'échantillons de matière.

**7.** Procédé selon l'une des revendications 1 à 6 **caractérisé en ce qu'**il consiste à comparer le quotient de fluorescence d'au moins un échantillon de matière, au quotient de fluorescence d'au moins un échantillon de produit de référence déterminé de la même manière que le quotient de fluorescence de l'échantillon de matière.

**8.** Procédé selon la revendication 7 **caractérisé en ce que** le forage étant effectué avec une injection de boue, l'échantillon de produit de référence est constitué par un échantillon de boue prélevé en cours de forage.

**9.** Procédé selon la revendication 8 **caractérisé en ce que** une valeur de quotient de fluorescence d'un échantillon de matière, proche de la valeur du quotient de fluorescence de l'échantillon de boue prélevé en cours de forage, est significative de l'absence d'hydrocarbures de formation dans l'échantillon de matière et une valeur différente, est significative d'une présence si de plus la valeur du flux d'émission de l'échantillon de matière est élevée.

**Patentansprüche**

**1.** Verfahren zum Nachweis und zur Charakterisierung von Kohlenwasserstoffen in unterirdischen Lagerstätten, umfassend die Durchführung folgender Stufen:

- im Verlauf einer Bohrung wird eine repräsentative Materialprobe von repräsentativem Material des durchquerten Gesteins entnommen,
- aus der Materialprobe wird eine bestimmte Materialmenge zur Analyse entnommen,

- die in der zu analysierenden Materialmenge enthaltenen Kohlenwasserstoffe werden mittels einer bekannten Menge eines Kohlenwasserstoff-Lösungsmittels zur Bildung eines anfänglichen Extrakts extrahiert,

**dadurch gekennzeichnet, dass** das Verfahren zusätzlich aus der Durchführung folgender Maßnahmen besteht:

- der anfängliche Extrakt wird mit einem Faktor d unter Erhalt eines endgültigen Extrakts verdünnt,
- der endgültige Extrakt wird mittels UV-Strahlen mit einer Wellenlänge, die im Bandbereich im wesentlichen von 250 bis 400 nm ausgewählt ist, angeregt,
- in einem Bandbereich L von Wellenlängen wird ein Emissionsspektrum aufgrund der Anregung des endgültigen Extrakts registriert,
- der Emissionsflusswert der Probe wird unter Anwendung der folgenden Formel berechnet:

$$FLUX = d * \sum_{i=1}^{n} I(\lambda i)$$

worin

- FLUX den Emissionsfluss der Materialprobe bedeutet,
- $I(\lambda i)$ die Intensität der Strahlung bedeutet, die bei einer Wellenlänge $\lambda i$ des Bandbereiches L durch den endgültigen Extrakt emittiert wird,
- n die Anzahl der Wellenlängen des Emissionsspektrums bedeutet, das in einem Abstand zwischen 1 und 20 nm einer Probennahme unterzogen worden ist, und L dividiert durch den Abstand bedeutet,
- i von 1 bis n variiert;
  wobei der Emissionsflusswert der Materialprobe proportional zur Konzentration an fluoreszierenden Elementen der Materialprobe und davon insbesondere der Kohlenwasserstoffe der unterirdischen Lagerstätte ist,

- der Quotient der Fluoreszenz der Materialprobe wird mittels folgender Formel berechnet:

$$QF = Fläche\ A\ /\ Fläche\ B$$

worin:

QF den Quotienten der Fluoreszenz der Materialprobe bedeutet,
die Flächen A und B gemäß folgenden Formeln berechnet werden:

$$Fläche\ A = \sum_{i=x+1}^{n} I(\lambda i)$$

$$Fläche\ B = \sum_{i=1}^{x} I(\lambda i)$$

worin:
- $I(\lambda i)$ und n den vorstehenden Definitionen entsprechen,
- x eine ganze Zahl von 1 bis n bedeutet, wobei der Wert des Quotienten der Fluoreszenz der Materialprobe repräsentativ für die Art der in der Materialprobe enthaltenen Kohlenwasserstoffe ist.

**2.** Verfahren nach Anspruch 1, **dadurch gekennzeichnet, dass** es sich beim Kohlenwasserstofflösungsmittel vorzugsweise um Cyclohexan handelt.

**3.** Verfahren nach Anspruch 1 oder 2, **dadurch gekennzeichnet, dass** der Bandbereich L von Wellenlängen vorzugsweise im wesentlichen im Bereich von 280 bis 500 nm liegt.

**4.** Verfahren nach den Ansprüchen 1, 2 oder 3, **dadurch gekennzeichnet, dass** der Wert x dem Bezugspunkt einer Wellenlänge $\lambda x$ entspricht, die im wesentlichen gleich 367 nm ist, der repräsentativ für den Cutoff zwischen den Spektren, die einerseits durch die monoaromatischen, diaromatischen und einen Teil der triaromatischen Kohlenwasserstoffe und andererseits durch die polyaromatischen Kohlenwasserstoffe emittiert werden.

**5.** Verfahren nach Anspruch 4, **dadurch gekennzeichnet, dass** ein geringer Wert des Quotienten der Fluoreszenz der Materialprobe ein Anzeichen für die Anwesenheit für die leichten Kohlenwasserstoffe in der Materialprobe darstellt und ein hoher Wert ein Anzeichen für die Anwesenheit von schweren Kohlenwasserstoffen darstellt.

**6.** Verfahren nach einem der Ansprüche 1 bis 5, **dadurch gekennzeichnet, dass** es aus folgenden Stufen besteht:

Entnehmen einer Reihe von Materialproben in unterschiedlichen Tiefen im Verlauf der Bohrung, Bestimmen des Quotienten der Fluoreszenz und des Emissionsflusses jeder Probe, anschließend Zeichnen von repräsentativen Kurven des Quotienten der Fluoreszenz und des Emissionsflusses in Abhängigkeit von der Tiefe, wobei die Variationen des Fluoreszenzquotienten und des Emissionsflusses jeweils eine Charakterisierung der Natur der fluoreszierenden Elemente und die Abschätzung der Bedeutung der Konzentration dieser Elemente in der Reihe von Materialproben ermöglichen.

**7.** Verfahren nach einem der Ansprüche 1 bis 6, **dadurch gekennzeichnet, dass** es aus einem Vergleich des Fluoreszenzquotienten mindestens einer Materialprobe mit dem Fluoreszenzquotienten mindestens einer Probe eines Referenzprodukts besteht, dessen Fluoreszenzquotient auf die gleiche Weise wie der Fluoreszenzquotient der Materialprobe bestimmt worden ist.

**8.** Verfahren nach Anspruch 7, **dadurch gekennzeichnet, dass** die Bohrung unter Injektion von Schlamm durchgeführt wird, wobei die Probe des Referenzprodukts aus einer im Laufe der Bohrung entnommenen Schlammprobe besteht.

**9.** Verfahren nach Anspruch 8, **dadurch gekennzeichnet, dass** ein Wert des Fluoreszenzquotienten einer Materialprobe, der nahe am Wert des Fluoreszenzquotienten der im Laufe der Bohrung entnommenen Schlammprobe liegt, ein Anzeichen für die Abwesenheit von Kohlenwasserstoffen in unterirdischen Formationen darstellt und ein davon abweichender Wert ein Anzeichen für die Anwesenheit darstellt, wenn zusätzlich der Wert des Emissionsflusses der Materialprobe erhöht ist.

**Claims**

**1.** Process for detecting and characterizing formation hydrocarbons which consists in carrying out the following steps:

- taking a sample of material which is representative of the rocks passed through during a drilling operation,
- taking a determined amount of material to be analysed from the said sample of material,
- extracting the hydrocarbons contained in the said amount of material to be analysed, using a known amount of a solvent for hydrocarbons, in order to make up an initial extract,

**characterized in that** it also consists in carrying out the following operations:

- diluting the initial extract by a factor d in order to obtain a final extract,
- exciting the final extract with ultraviolet radiation of a wavelength chosen within the band substantially between 250 and 400 nanometres,
- recording, in a wavelength band L, an emission spectrum resulting from the excitation of the final extract,
- calculating the emission flux of the sample of material by applying the following formula:

$$FLUX = d * \sum_{i=1}^{n} I(\lambda i)$$

in which

- FLUX represents the emission flux of the sample of material
- $I(\lambda i)$ represents the intensity of the radiation emitted at a wavelength $\lambda i$ of the band L by the final extract,
- n represents the number of wavelengths of the emission spectrum sampled at a pitch of between 1 and 20 nanometres, and is equal to L divided by the pitch,
- i ranges from 1 to n,

    the value of the emission flux of the sample of material is proportional to the concentration of fluorescent elements in the sample of material, including, in particular, the formation hydrocarbons,

- calculating the fluorescence quotient of the sample of material using the following formula:

FQ = area A / area B

in which:

FQ represents the fluorescence quotient of the sample of material,
area A and area B are calculated, respectively, by the following formulae:

$$Area\ A = \sum_{i=x+1}^{n} I(\lambda i)$$

$$Area\ B = \sum_{i=1}^{x} I(\lambda i)$$

in which:

- $I(\lambda i)$ and n are defined as above,
- x is an integer between 1 and n,

the value of the fluorescence quotient of the sample of material is representative of the nature of the hydrocarbons contained in the sample of material.

2. Process according to Claim 1, **characterized in that** the solvent for hydrocarbons is preferably cyclohexane.

3. Process according to Claim 1 or 2, **characterized in that** the wavelength band L is preferably substantially between 280 and 500 nanometres.

4. Process according to Claim 1, 2 or 3, **characterized in that** the value x corresponds to the reference point of a wavelength $\lambda x$ substantially equal to 367 nanometres, which is representative of the cutoff between the spectra emitted, on the one hand, by the monoaromatic and biaromatic hydrocarbons and by a portion of the triaromatic hydrocarbons, and, on the other hand, by the polyaromatic hydrocarbons.

5. Process according to Claim 4, **characterized in that** a low value for the fluorescence quotient of the sample of material indicates the presence of light hydrocarbons in the said sample and a high value indicates the presence of heavy hydrocarbons.

6. Process according to one of Claims 1 to 5, **characterized in that** it consists in taking a series of samples of material

**EP 0 927 886 B1**

at different depths during the drilling, in determining the fluorescence quotient and the emission flux of each sample, and then in plotting the curves representing the said fluorescence quotient and the said emission flux as a function of the depths, of the variations of the fluorescence quotient and of the emission flux, thus making it possible, respectively, to characterize the nature of the fluorescent elements and to assess the degree of concentration of the said elements in the series of samples of material.

7. Process according to one of Claims 1 to 6, **characterized in that** it consists in comparing the fluorescence quotient of at least one sample of material with the fluorescence quotient of at least one sample of reference product determined in the same way as the fluorescence quotient of the sample of material.

8. Process according to Claim 7, **characterized in that**, since the drilling is carried out with an injection of mud, the sample of reference product consists of a sample of mud taken during the drilling.

9. Process according to Claim 8, **characterized in that** a fluorescence quotient value for a sample of material which is close to the value of the fluorescence quotient for the sample of mud taken during the drilling indicates the absence of formation hydrocarbons in the sample of material, and a different value indicates a presence, if, zurthermore, the value of the emission flux for the sample of material is high.

FIG.1

FIG.2

FIG.3

FIG.4